(19) Europäisches Patentamt
European Patent Office
Office européen des brevets

(11) Veröffentlichungsnummer : **0 030 015**
**B1**

(12) # EUROPÄISCHE PATENTSCHRIFT

(45) Veröffentlichungstag der Patentschrift : **21.03.84**

(21) Anmeldenummer : **80107428.7**

(22) Anmeldetag : **27.11.80**

(51) Int. Cl.³ : **C 07 D487/04**, C 07 D249/04, C 07 D403/06, C 07 C 69/78, A 61 K 31/55// C07D209/48, C07C49/786, C07C33/24, (C07D487/04, 249/00, 223/00)

(54) **Triazolobenzazepine, Zwischenprodukte und Verfahren zu ihrer Herstellung, sowie diese enthaltende Präparate.**

(30) Priorität : **30.11.79 US 99109**

(43) Veröffentlichungstag der Anmeldung : **10.06.81 Patentblatt 81/23**

(45) Bekanntmachung des Hinweises auf die Patenterteilung : **21.03.84 Patentblatt 84/12**

(84) Benannte Vertragsstaaten : **BE CH DE FR GB IT LI NL SE**

(56) Entgegenhaltungen :
**DE-A- 1 955 349**
**DE-A- 2 055 889**
**DE-A- 2 524 048**
**US-A- 3 717 653**

(73) Patentinhaber : **F. HOFFMANN-LA ROCHE & CO. Aktiengesellschaft**
**CH-4002 Basel (CH)**

(72) Erfinder : **Fryer, Rodney Ian**
**Eton Drive 5**
**North Caldwell, N.J. (US)**
Erfinder : **Trybulski, Eugene J.**
**Homer Street 13**
**Parsippany, N.J. (US)**
Erfinder : **Walser, Armin**
**Crane Avenue 19**
**West Caldwell, N.J. (US)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 030 015 B1

# Triazolobenzazepine, Zwischenprodukte und Verfahren zu ihrer Herstellung, sowie diese enthaltende Präparate

Die vorliegende Erfindung betrifft Triazolobenzazepine der allgemeinen Formel

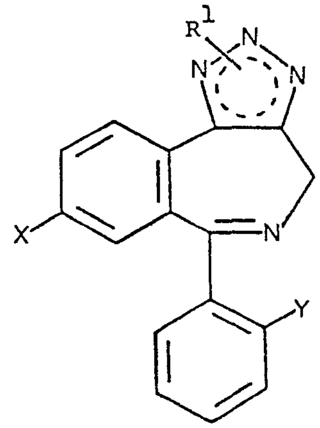

(I)

worin $R^1$ Wasserstoff oder niederes Alkyl, X und Y je Wasserstoff oder Halogen mit einer Atomnummer von höchstens 35 und die gestrichelte Linie 2 zusätzliche Bindungen im Triazolring bedeuten, und pharmazeutisch annehmbare Säureadditionssalze davon.

In DE-Al-2.524.048 sind Pyrazolo[4,3-d][2]benzazepine mit antidepressiven und anxiolytischen Eigenschaften beschrieben. In US-A-3.717.653 sind Tetrazolo[1,5-a][1,4]benzodiazepine mit sedativen und beruhigenden Eigenschaften beschrieben. In DE-Al-1.955.349 sind s-Triazolo[4,3-a][1,4]benzodiazepine mit muskelrelaxierenden, beruhigenden und dämpfenden Eigenschaften beschrieben.

Ganz besonders bevorzugte Verbindungen sind solche, worin X Chlor, Y Wasserstoff, Chlor oder Fluor und $R^1$ Wasserstoff oder Methyl bedeuten, wobei für den Methylrest die 2- und die 3-Stellung bevorzugt ist.

Repräsentative Vertreter von bevorzugten Verbindungen der allgemeinen Formel I sind :

8-Chlor-6-phenyl-2H,4H-[1,2,3]triazolo[4,5-d][2]benzazepin,
8-Chlor-6-(2-fluorphenyl)-2H,4H-[1,2,3]triazolo[4,5-d][2]benzazepin und
8-Chlor-6-(2-chlorphenyl)-2H,4H-[1,2,3]triazolo[4,5-d][2]benzazepin.

Der Ausdruck « niederes Alkyl » bezeichnet geradkettige oder verzweigte, gesättigte Kohlenwasserstoffreste mit 1 bis 7, vorzugsweise 1 bis 4 Kohlenstoffatomen, wie Methyl, Äthyl, Isopropyl, Butyl und dergleichen.

Der Ausdruck « Halogen », wie er in der vorliegenden Beschreibung verwendet wird, bedeutet die drei Formen Chlor, Brom und Fluor.

Die Triazolobenzazepine der allgemeinen Formel I und ihre pharmazeutisch annehmbaren Säureadditionssalze können erfindungsgemäss dadurch hergestellt werden, dass man eine Verbindung der allgemeinen Formel

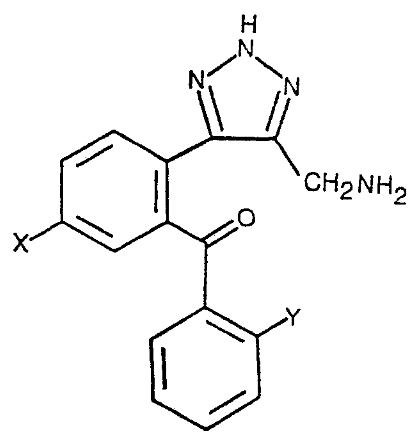

(II)

worin X und Y obige Bedeutung besitzen, cyclisiert, erwünschtenfalls eine so erhaltene Verbindung der allgemeinen Formel

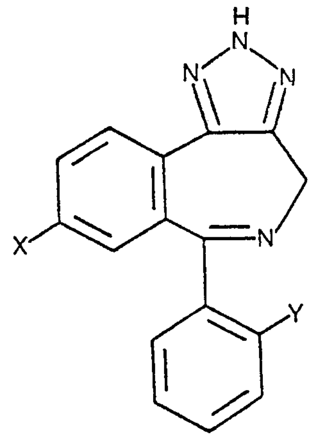

(Ia)

worin X und Y obige Bedeutung besitzen, entsprechend alkyliert, und/oder erwünschtenfalls eine Verbindung der allgemeinen Formel I in ein pharmazeutisch annehmbares Säureadditionssalz überführt.

Das erfindungsgemässe Verfahren und die Herstellung der benötigten Ausgangsstoffe wird durch die folgenden Reaktionsschemata illustriert :

Reaktionsschema 1

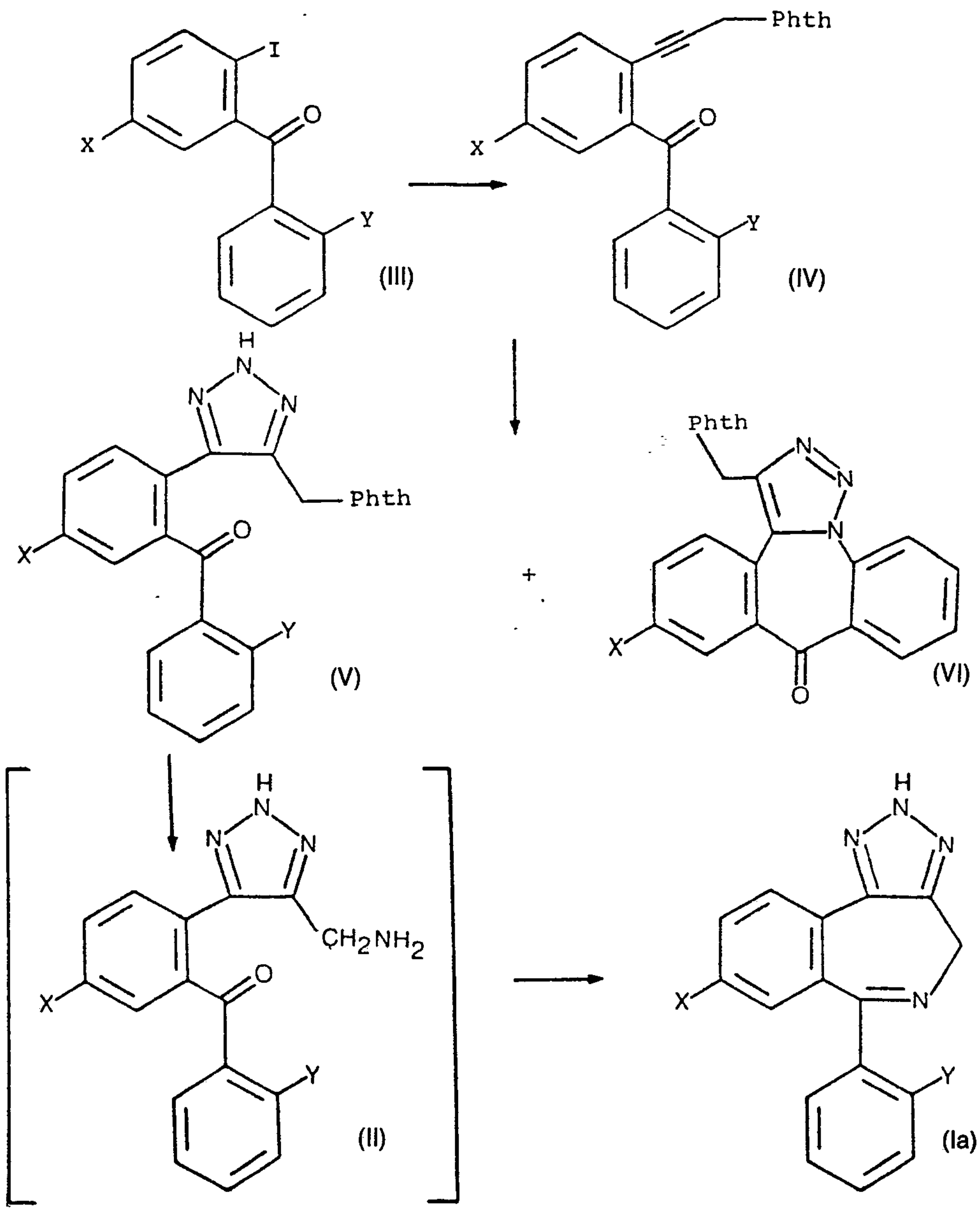

(III) (IV) (V) + (VI) (II) (Ia)

Reaktionsschema 2

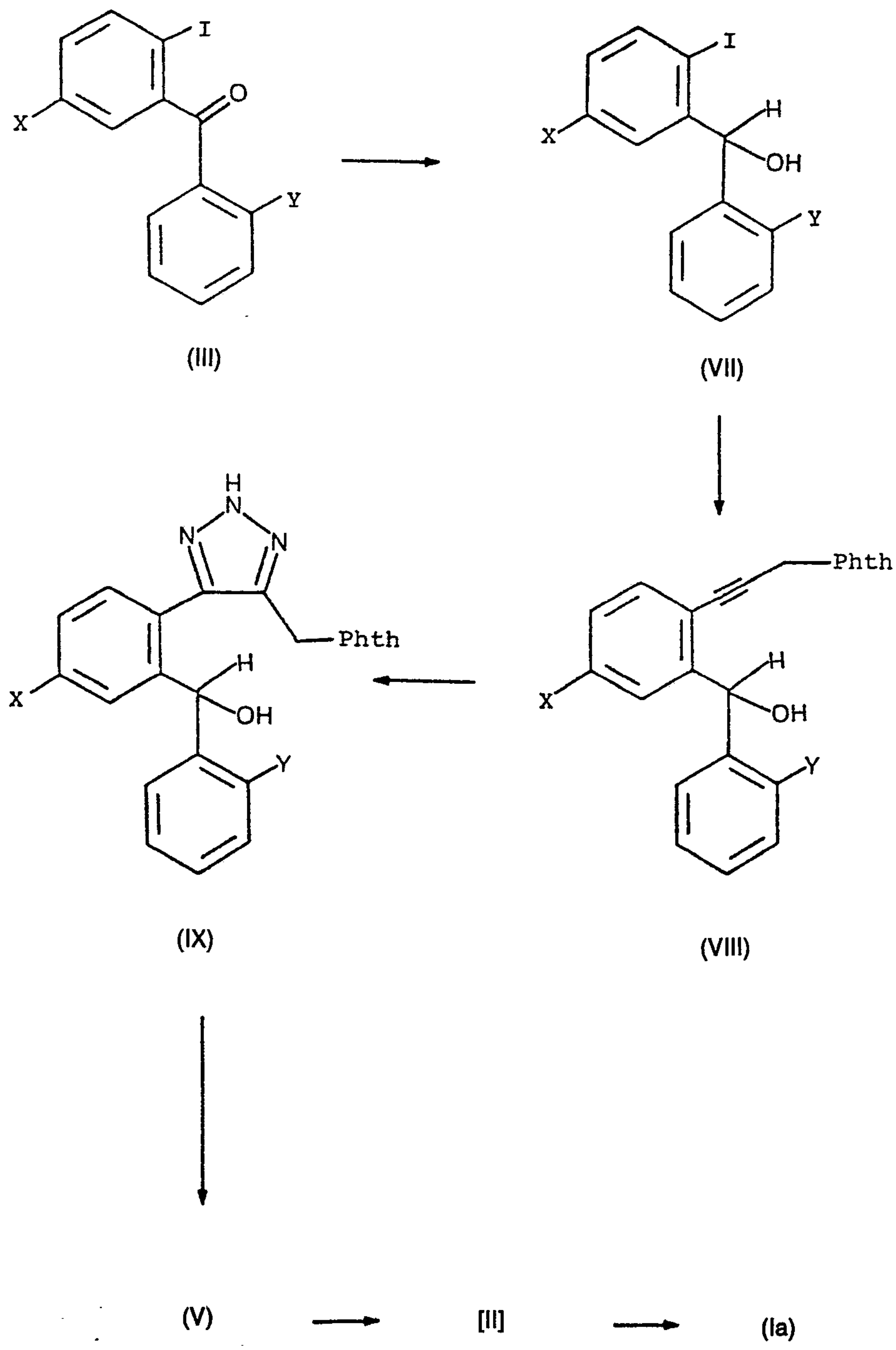

(III) (VII) (VIII) (IX)

(V) [II] (Ia)

Reaktionsschema 3

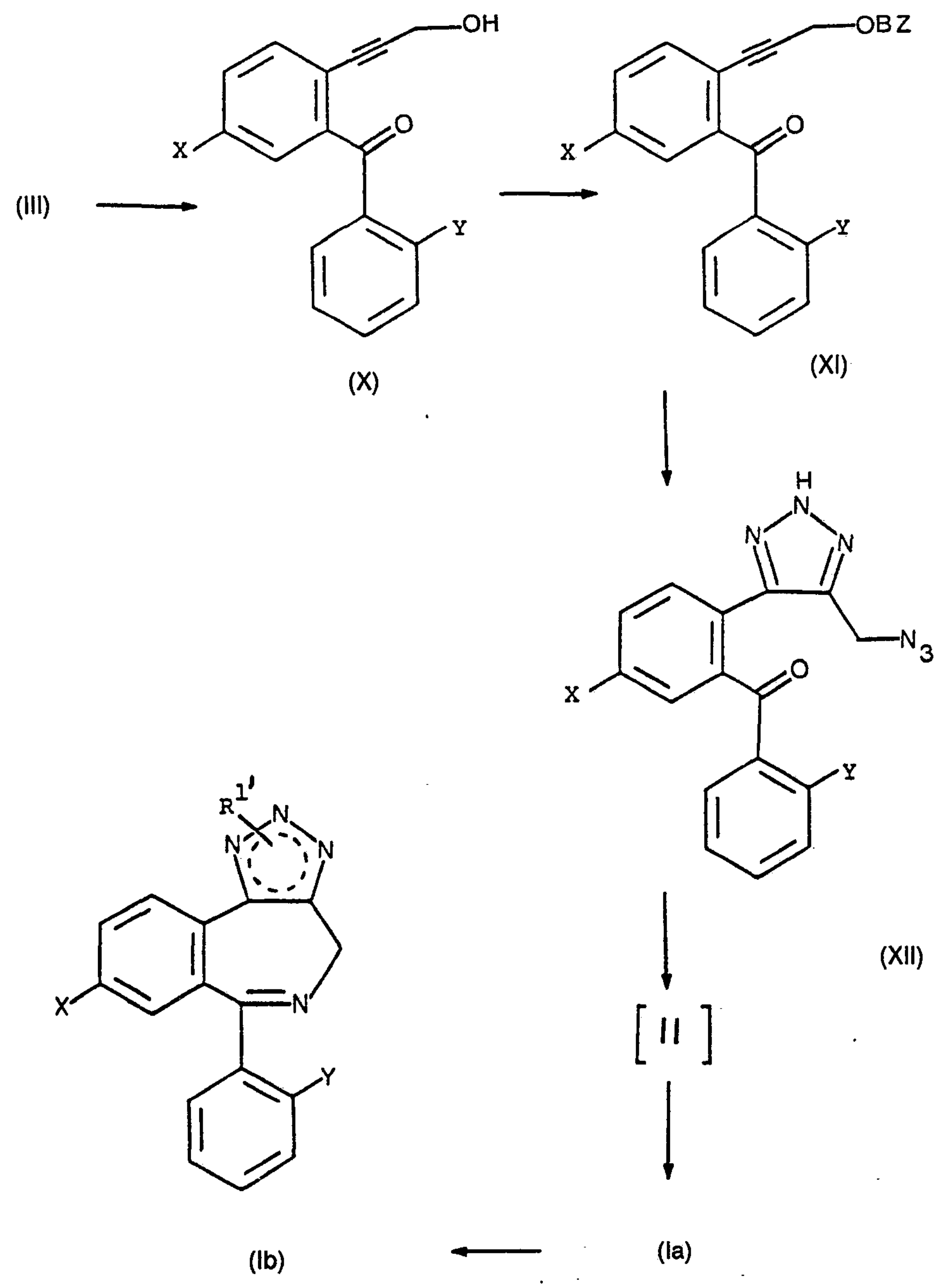

(III) (X) (XI) (XII) [II] (Ia) (Ib)

worin X, Y und die gestrichelte Linie obige Bedeutung besitzen $R^{1'}$ niederes Alkyl, Phth Phthalimido und BZ Benzoyl bedeuten.

Die Verbindungen der allgemeinen Formeln II, V, IX und XII sind ebenfalls Gegenstand der vorliegenden Erfindung.

Reaktionsschema 1

III ⟶ IV

Eine Verbindung der Formel IV kann hergestellt werden, indem man eine Verbindung der Formel III mit Propargylphthalimid umsetzt, und zwar in Gegenwart eines Palladium(II)-Salzes, wie Palladium(II) chlorid oder -Acetat, eines Organophosphins, wie beispielsweise Triphenylphosphin, von Kupfer(I)jodid und eines tertiären oder, vorzugsweise, eines sekundären Amins, wie Diäthylamin oder Diisopropylamin. Als Lösungsmittel kommen das eingesetzte Amin selber, z. B. Diäthylamin, halogenierte Kohlenwasserstoffe, z. B. Methylenchlorid, Dimethylformamid oder Aether in Frage. Die Reaktionstemperatur kann in einem Bereich von 0 °C bis 70 °C variieren, wobei Raumtemperatur bevorzugt wird. Wird die Reaktion bei

Raumtemperatur oder darunter durchgeführt, so ist die Anwesenheit von Kupfer(I)jodid zwingend ; dies ist nicht der Fall, wenn die Reaktion unter Erwärmen durchgeführt wird. Die Anwesenheit eines Organophosphins ist nicht absolut notwendig, aber sehr vorteilhaft. An Stelle eines Palladium(II)-Salzes und eines Organophosphins kann man auch einen geeigneten Komplex, wie das Dichlor-bis(triphenylphosphin)Palladium(II), verwenden.

Das Ausgangsmaterial der Formel III kann hergestellt werden, indem man das entsprechende, bekannte Aminobenzophenon mit Natriumnitrit in Schwefelsäure diazotiert, das entsprechende Diazoniumsalz als Tetrafluoroborat ausfällt, dieses in Wasser aufschlämmt und durch Behandeln mit Kaliumjodid ins entsprechende Jodbenzophenon überführt. Diese Reaktionen werden nach an sich bekannten Methoden durchgeführt.

IV → V und VI

Eine Verbindung der Formel IV kann mit einem Alkalimetallazid, wie Natrium- oder Kaliumazid und einer niederen Fettsäure, wie Essigsäure und Propionsäure, in einem polaren aprotischen Lösungsmittel, wie Dimethylsulfoxid oder Dimethylformamid, umgesetzt werden. Die Reaktionstemperatur kann dabei von Raumtemperatur bis 110 °C variiert werden, wobei die besonders bevorzugte Temperatur von der Art der Substituenten X und Y abhängig ist. Das Nebenprodukt der Formel VI wird anschliessend abgetrennt und verworfen.

V → II → Ia

Eine Verbindung der Formel V kann anschliessend mit. einer wässrigen Lösung eines niederen Alkylamins, beispielsweise Methylamin umgesetzt werden. Als Lösungsmittel verwendet man einen $C_1$ bis $C_4$ Alkohol, vorzugsweise Aethanol. Die Reaktion wird vorzugsweise bei etwa Raumtemperatur durchgeführt. Das in einem ersten Schritt gebildete offenkettige Amin der Formel II, das nicht isoliert wird, cyclisiert spontan zum Endprodukt der Formel Ia. Das Endprodukt wird anschliessend durch bekannte Filtriertechniken isoliert.

Andererseits ist es auch möglich eine Verbindung der Formel Ia dadurch herzustellen, dass man eine Verbindung der Formel V mit Hydrazin in einem inerten Lösungsmittel, wie Aethanol, einer Mischung aus Aethanol und Chloroform, Tetrahydrofuran oder wässrigem Aethanol, umsetzt. Die Reaktionstemperatur kann in einem Bereich von Raumtemperatur bis 100 °C variieren, wobei die Rückflusstemperatur des gewählten Lösungsmittels bevorzugt wird. Das entstandene Produkt wird mit verdünnter Mineralsäure ausgeschüttelt und anschliessend durch Neutralisation zurückgewonnen.

Eine dritte Methode welche verwendet werden kann, um Verbindungen der Formel Ia herzustellen, umfasst eine basische und anschliessend eine saure Hydrolyse einer Verbindung der Formel V. Für die saure Hydrolyse kann man eine 30-proz. Lösung einer Mineralsäure, wie Salzsäure, Bromwasserstoffsäure, Schwefelsäure oder Phosphorsäure verwenden. Die Reaktion wird bei der Siedetemperatur des Gemisches oder wenig darunter durchgeführt. Für die basische Hydrolyse kann man ein Alkalimetallhydroxid, wie Kalium- oder Natriumhydroxid verwenden. Um die Reaktanden in Lösung zu bringen, können inerte organische Lösungsmittel, wie die oben erwähnten, verwendet werden. Die Reaktion wird bei der Siedetemperatur des gewählten Lösungsmittels oder wenig darunter durchgeführt.

Reaktionsschema 2

III → VII

Eine Verbindung der Formel III kann mit einem Metallhydrid-Reduktionsmittel, wie Natriumborhydrid, in einem alkoholischen Lösungsmittel, wie Aethanol oder Methanol, oder Lithiumaluminiumhydrid in einer ätherischen Lösung, wie Tetrahydrofuran oder Dioxan, reduziert werden. Die Reaktionstemperatur liegt in einem Bereich von − 10 °C bis Raumtemperatur, wobei 0 °C bevorzugt wird.

VII → VIII

Eine Verbindung der Formel VII wird anschliessend mit einer Mischung aus Palladium(II)chlorid, Kupfer(I)jodid, Triphenylphosphin und Propargylphthalimid in einem Lösungsmittel, wie Methylenchlorid oder Dimethylformamid, umgesetzt ; und zwar in Gegenwart einer Base, z. B. ein Di- oder Tri-Alkylamin, wie beispielsweise Diäthylamin und Triäthylamin. Die obige Reaktion kann in einem Temperaturbereich von 0 °C bis 40 °C, vorzugsweise bei etwa Raumtemperatur, durchgeführt werden.

VIII → IX

Eine Verbindung der Formel VIII kann anschliessend mit einem Alkalimetallazid und einer niederen Fettsäure in der Gegenwart eines inerten polaren aprotischen Lösungsmittels, wie Dimethylsulfoxid und Dimethylformamid, umgesetzt werden. Die Reaktionstemperatur liegt in einem Bereich von 60 °C bis 100 °C, vorzugsweise bei etwa 90 °C.

IX ⟶ V

Ein Benzhydrol der Formel IX kann anschliessend mit einem Oxidationsmittel, wie dem Jones- oder Collins-Reagens, in einem inerten organischen Lösungsmittel, wie Methylenchlorid oder Aceton, oxidiert werden. Die Reaktionstemperatur kann in einem Bereich von − 10 °C bis Raumtemperatur variiert werden, wobei man vorzugsweise bei etwa 0 °C arbeitet.

Reaktionsschema 3

III ⟶ X

Eine Verbindung der Formel III kann mit Propargylalkohol in Gegenwart von Palladium(II)chlorid, Kupfer(I)-jodid, eines Organophosphins, z. B. Triphenylphosphin, und eines sekundären Amins, wie Diäthylamin oder Diisopropylamin, umgesetzt werden. Es können die gleichen Lösungsmittel und Reaktionsbedingungen wie für die Stufe III ⟶ IV verwendet werden.

X ⟶ XI

Eine Verbindung der Formel X kann mit einem Benzoylierungsmittel, wie Benzoylchlorid in Pyridin oder Benzosäureanhydrid in Pyridin, umgesetzt werden. Geeignete Lösungsmittel umfassen Pyridin oder Methylenchlorid. Die Reaktionstemperatur kann in einem Bereich von 0 °C bis Raumtemperatur liegen, wobei Raumtemperatur bevorzugt wird.

XI ⟶ XII

Eine Verbindung der Formel XI kann anschliessend mit einem Alkalimetallazid, wie Natrium- oder Kaliumazid in Gegenwart eines inerten polaren aprotischen Lösungsmittels, wie Dimethylformamid oder Dimethylsulfoxid, umgesetzt werden. Die Reaktion kann in einem Temperaturbereich von Raumtemperatur bis 100 °C, vorzugsweise bei etwa 80 °C, durchgeführt werden.

XII ⟶ II ⟶ Ia

Eine Verbindung der Formel XII kann anschliessend in Gegenwart eines Uebergangsmetallkatalysators, wie Raney-Nickel oder Platinoxid, und erwünschtenfalls in Gegenwart einer Mineralsäure, wie Salzsäure, hydriert, werden. Die Reaktion kann bei etwa Raumtemperatur und bei Drucken von Normaldruck bis 40 psi ($2{,}75 \cdot 10^5$ Pascal), vorzugsweise Atmosphärendruck, durchgeführt werden. Das in einem ersten Schritt gebildete offenkettige Amin der Formel II, das nicht isoliert wird, cyclisiert spontan zu einer Verbindung der Formel Ia.

Ia ⟶ Ib

Eine Verbindung der Formel Ia kann anschliessend mit einem Alkalimetallhydroxid, z. B. Natrium- oder Kaliumhydroxid, in einem $C_1$ bis $C_6$ alkohol, vorzugsweise in einem $C_1$ bis $C_4$ Alkohol, behandelt werden. Anschliessend kann die erhaltene Mischung mit einem geeigneten Alkylierungsmittel, wie Dialkylsulfat, z. B. Dimethylsulfat, einem Alkylhalogenid, wie Methylchlorid, oder einem Alkyltosylat, umgesetzt werden. Die Reaktion kann in einem Bereich von 0 °C bis Raumtemperatur durchgeführt werden, wobei Raumtemperatur bevorzugt wird.

Dem Hauptprodukt aus obiger Alkylierung wurde die Struktur des in 2-Stellung alkylierten Produktes, und dem Nebenprodukt die Struktur des in 3-Stellung alkylierten Produktes zugeordnet.

Der Ausdruck « pharmazeutisch annehmbare Säureadditionssalze » umfasst Salze sowohl mit anorganischen als auch mit organischen pharmazeutisch annehmbaren starken Säuren, wie Schwefelsäure, Salzsäure, Salpetersäure, Methansulfonsäure und p-Toluolsulfonsäure. Solche Salze können im Hinblick auf den Stand der Technik und die Natur der in Salze zu überführenden Verbindungen von jedem Fachmann sehr leicht hergestellt werden.

Die Triazolobenzazepine der Formel I und ihre pharmazeutisch annehmbaren Säureadditionssalze sind nützlich als Heilmittel und zeichnen sich durch anxiolytische, sedative, muskelrelaxierende und anticonvulsive Aktivitäten aus. Diese Verbindungen können in Form üblicher pharmazeutischer Zubereitungen verwendet werden ; beispielsweise können sie mit üblichen organischen oder anorganischen, inerten, für parenterale oder enterale Verabreichung geeigneten Trägerstoffen vermischt werden, beispielsweise mit Wasser, Gelatine, Milchzucker, Stärke, Magnesiumstearat, Talk, pflanzliche Oele, Gummi arabicum, Polyalkylenglykolen, Vaselin oder dergleichen. Sie können als übliche pharmazeutische Formen verabreicht werden, beispielsweise als feste Formen, wie Tabletten, Dragées, Kapseln, Suppositorien oder dergleichen, oder als flüssige Formen, wie Lösungen, Suspensionen oder Emulsionen. Darüberhinaus können die erfindungsgemässe Verbindungen enthaltenden pharmazeutischen Zubereitungen üblichen pharmazeutischen Operationen, wie Sterilisieren, unterzogen werden, und sie

können übliche pharmazeutische Hilfsstoffe enthalten, wie Konservierungsmittel, Stabilisierungsmittel, Netzmittel, Emulgiermittel, Salze zur Veränderung des osmotischen Druckes oder Puffer. Die pharmazeutischen Präparate können auch noch andere therapeutisch wertvolle Stoffe enthalten.

Die nachfolgende Tabelle zeigt die Resultate, welche erhalten wurden, als die nachfolgend aufgezählten Verbindungen bestimmten, wohlbekannten Versuchen, wie dem Test an der geneigten Ebene, dem Kampftest, dem Test an der unanästhesierten Katze und dem Antipentamethylentetrazoltest (Metrazoltest) unterzogen wurden, ebenso auch Angaben über ihre Toxizität :

A ; 8-Chlor-6-(2-fluorphenyl)-2H,4H-[1,2,3]triazolo[4,5-d][2]benzazepin-methansulfonat
B : 8-Chlor-6-(2-chlorphenyl)-2H,4H-[1,2,3]triazolo[4,5-d][2]benzazepin-methansulfonat
C : 8-Chlor-6-phenyl-2H,4H-[1,2,3]triazolo[4,5-d][2]benzazepin

(Siehe Tabelle Seite 9 f.)

Tabelle

| Verbindung | Geneigte Ebene Maus ($ED_{50}$) | Kampftest Maus (wirksame Dosis) | Unanästh. Katze (kleinste wirksame Dosis) | Metrazol Maus ($ED_{50}$) | Toxizität Maus ($DL_{50}$) |
|---|---|---|---|---|---|
| A | 302 mg/kg p.o. | 50 mg/kg p.o. | 10 mg/kg p.o. | 2.1 mg/kg p.o. | $>$1000 mg/kg p.o. |
| B | 400 mg/kg p.o. | 100 mg/kg p.o. | 10 mg/kg p.o. | 2.6 mg/kg p.o. | $>$1000 mg/kg p.o. |
| C | 400 mg/kg p.o. | 100 mg/kg p.o. | 10 mg/kg p.o. | | |

Eine geeignete pharmazeutische Dosierungseinheit kann zwischen 1 bis 500 mg einer Verbindung der Formel I oder eines pharmazeutisch annehmbaren Säureadditionssalzes davon enthalten, wobei ein Dosierungsbereich von 1 mg bis 100 mg für orale Verabreichung und ein Dosierungsbereich von 1 mg bis 50 mg für parenterale Verabreichung bevorzugt ist. In jedem Einzelfall muss jedoch die Person, welche die Verabreichung der fraglichen Verbindungen durchführt oder überwacht, die Dosierung nach ihrem fachlichen Urteil den individuellen Erfordernissen anpassen. Es ist zu beachten, dass die vorstehenden Dosierungen lediglich im Sinn von Beispielen angegeben sind und dass sie den Umfang und die Ausführung der vorliegenden Erfindung in keiner Weise einschränken.

Der Ausdruck « Dosierungseinheit » wie er in der vorliegenden Beschreibung verwendet wird, bezeichnet einzelne pharmazeutische Einheiten, welche sich als Einheitsdosen für Säuger eignen und von welchen jede eine vorbestimmte Menge des Wirkstoffes enthält, welche so berechnet wurde, dass die zusammen mit dem erforderlichen pharmazeutischen Verdünnungsmittel, Träger oder Vehikel den gewünschten therapeutischen Effekt zur Folge hat. Die nachfolgenden Beispiele sollen die Erfindung illustrieren, jedoch nicht einschränken. Alle Temperaturen sind in Celsius Graden angegeben, sofern nichts anderes vermerkt ist.

Beispiel 1

Eine Mischung aus 20 g (0,05 Mol) 1-[4-chlor-2-benzoylphenyl]-3-phthalimidopropine, 15,0 g (0,23 Mol) Natriumazid und 3,0 ml (0,05 Mol) Essigsäure in 300 ml Dimethylsulfoxid wird während 3 Tagen auf 90° erwärmt. Die Reaktionsmischung wird abgekühlt und mit 1,5 Liter Wasser und 250 ml Methylenchlorid verdünnt. Nach Ausschütteln der Mischung mit Aether werden die vereinigten ätherischen Auszüge mit Wasser gewaschen, über Natriumsulfat getrocknet und im Vakuum eingedampft. Durch Umkristallisieren des Rohproduktes aus Methylenchlorid/Aether erhält man 4-[4-Chlor-2-benzoylphenyl]-5-[-phthalimidomethyl]-2H-1,2,3-triazol als farblose Nadeln vom Smp. 134-136°.

Beispiel 2

Eine Mischung aus 10 g (24 mMol) 1-[4-Chlor-2-(2-fluorbenzoyl)phenyl]-3-phthalimidopropin, 7,5 g (115 mMol) Natriumazid, 2,2 ml (36 mMol) Essigsäure und 150 ml Dimethylsulfoxid wird während 48 Stunden auf 50° erwärmt. Die Reaktionsmischung wird abgekühlt und mit Wasser verdünnt. Der entstandene Niederschlag wird abfiltriert, wobei 11,0 g einer rohen Mischung anfallen. Nach Reinigung durch Säulenchromatographie (Kieselgel, 105 g ; Elutionsmittel : Methylenchlorid/Aether (0-5 %)-Gradient) erhält man als erste Hauptkomponente feine Prismen vom Smp. 252-253° und als zweite Komponente 4-[4-Chlor-2-(2-fluorbenzoyl)phenyl]-5-[N-phtalimidomethyl]-2H-1,2,3-triazol als lichtempfindliche Prismen vom Smp. 147-148° (Schaumbildung).

Beispiel 3

Eine Mischung aus 6,8 g (15,6 mMol) 1-[4-Chlor-2-(2-chlorbenzoyl)phenyl]-3-phthalimidopropin, 5,0 g (85 mMol) Natriumazid 1,0 ml (17 mMol) Essigsäure und 75 ml Dimethylsulfoxid wird während 48 Stunden auf 50° erwärmt. Die Reaktionsmischung wird abgekühlt, mit Wasser verdünnt und mit Methylenchlorid ausgeschüttelt. Die Methylenchlorid-Phase wird mit Wasser gewaschen, über Natriumsulfat getrocknet und im Vakuum eingedampft, wobei 6,5 g eines gelben Schaumes anfallen. Nach Reinigung durch Säulenchromatographie (Kieselgel, 40 g ; Elutionsmittel : Methylenchlorid/Aether (0-5 %)-Gradient) erhält man 4-[4-Chlor-2-(2-chlorbenzoyl)phenyl]-5-[N-phthalimidomethyl]-2H-1,2,3-triazol als farblose Prismen vom Smp. 186-187°.

Beispiel 4

Eine Mischung aus 10,0 g (25 mMol) 1-[2-(2-Chlorbenzoyl)phenyl]-3-phthalimidopropin, 7,5 g (115 mMol) Natriumazid, 1,5 ml (25 mMol) Essigsäure und 150 ml Dimethylsulfoxid wird während 24 Stunden auf 90° erwärmt. Die Reaktionsmischung wird abgekühlt, mit Wasser verdünnt und mit Methylenchlorid ausgeschüttelt. Die Methylenchlorid-Phase wird mit Wasser gewaschen, über Natriumsulfat getrocknet und im Vakuum eingedampft, wobei ein gelbes Oel anfällt. Durch Behandeln des Rückstandes mit einer Mischung aus Aether und Petroläther erhält man 4-[2-(2-Chlorbenzoyl)phenyl]-5-[N-phthalimidomethyl]-2H-1,2,3-triazol als crèmefarbene Prismen vom Smp. 105-106° (Schaumbildung).

Beispiel 5

Eine Mischung aus 10,0 g (27,0 mMol) 1-(2-Benzoylphenyl)-3-phthalimidopropin, 7,5 g (115 mMol) Natriumazid, 1,5 ml (25 mMol) Essigsäure und 150 ml Dimethysulfoxid wird während 60 Stunden auf 90° erwärmt. Die Reaktionsmischung wird abgekühlt, mit Wasser verdünnt und mit Methylenchlorid ausgeschüttelt. Die Methylenchlorid-Phase wird mit Wasser gewaschen, über Natriumsulfat getrocknet und im Vakuum eingedampft, wobei ein braunes Oel anfällt. Aus Aether erhält man 4-[2-Benzoylphenyl]-5-[N-phthalimidomethyl]-2H-1,2,3-triazol als bräunliche Kristalle. Durch Umkristallisieren aus Aether/Methylenchlorid erhält man weisse Prismen vom Smp. 204-205°.

Beispiel 6

Eine Mischung aus 6,0 g (13,6 mMol) 4-[4-Chlor-2-benzoylphenyl]-5-[N-phthalimidomethyl]-2H-1,2,3-triazol, 50 ml 40-proz. wässriges Methylamin und 100 ml Aethanol (95 %) wird während 12 Stunden bei Raumtemperatur gerührt. Nach Entfernen der Lösungsmittel unter vermindertem Druck wird der Rückstand mit einer Mischung aus Aether/Methylenchlorid (2 : 1) behandelt. Nach Abfiltrieren des entstandenen Niederschlages wird das Filtrat mit Wasser gewaschen und über Natriumsulfat getrocknet. Nach Eindampfen der ätherischen Lösung im Vakuum erhält man 8-Chlor-6-phenyl-2H,4H-[1,2,3]triazolo[4,5-d][2]benzazepin als farblose Prismen vom Smp. 199-200°.

Beispiel 7

In Analogie zur Herstellung von 8-Chlor-6-phenyl-2H,4H-[1,2,3]triazolo[4,5-d][2]benzazepin erhält man 8-Chlor-6-(2-fluorphenyl)-2H,4H-[1,2,3]triazolo[4,5-d][2]benzazepin als farblosen Festkörper Smp. 192-193°.

Aus einer Lösung äquimolarer Mengen obiger Verbindung und Methansulfonsäure in Methanol erhält man, durch Ausfällen mit Aether, 8-Chlor-6-(2-fluorphenyl)-2H,4H-[1,2,3]triazolo[4,5-d][2]benzazepin-methansulfonat. Durch Umkristallisieren aus Methanol/Aether erhält man das Methansulfonat als gelbe Prismen vom Smp. 237-238°.

Beispiel 8

In Analogie zur Herstellung von 8-Chlor-6-phenyl-2H,4H-[1,2,3]triazolo[4,5-d][2]benzazepin erhält man 8-Chlor-6-(2-chlorphenyl)-2H,4H-[1,2,3]triazolo[4,5-d][2]benzazepin als farblose Prismen vom Smp. 176-177°.

Aus einer Lösung äquimolarer Mengen obiger Verbindung und Methansulfonsäure in Methanol erhält man, durch Ausfällen mit Aether, 8-Chlor-6-(2-chlorphenyl)-2H,4H-[1,2,3]triazolo[4,5-d][2]benzazepin-methansulfonat. Durch Umkristallisieren aus Methanol/Aether erhält man das Methansulfonat als gelbe Prismen vom Smp. 236-237°.

Beispiel 9

In Analogie zur Herstellung von 8-Chlor-6-phenyl-2H,4H-[1,2,3]triazolo[4,5-d][2]benzazepin erhält man 6-(2-Chlorphenyl)-2H,4H-[1,2,3]triazolo[4,5-d][2]benzazepin als farblose Prismen vom Smp. 191-193°.

Aus einer Lösung äquimolarer Mengen der obigen Verbindung und Methansulfonsäure in Methanol erhält man, durch Ausfällen mit Aether, 6-(2-Chlorphenyl)-2H,4H-[1,2,3]triazolo[4,5-d]benzazepin-methansulfonat. Durch Umkristallisieren aus Methanol/Aether erhält man das Methansulfonat als gelbe Nadeln vom Smp. 238-240°.

Beispiel 10

In Analogie zur Herstellung von 8-Chlor-6-phenyl-2H,4H-[1,2,3]triazolo[4,5-d][2]benzazepin erhält man 6-Phenyl-2H,4H-[1,2,3]triazolo[4,5-d][2]benzazepin als farblose Prismen vom Smp. 205-207°.

Aus einer Lösung äquimolarer Mengen obiger Verbindung und Methansulfonsäure erhält man, durch Ausfällen mit Aether, 6-Phenyl-2H,4H-[1,2,3]triazolo[4,5-d][2]benzazepinmethansulfonat. Durch Umkristallisieren aus Methanol/Aether erhält man das Methansulfonat als gelbe Prismen vom Smp. 169-170°.

Beispiel 11

Eine Mischung aus 1,5 g (3,5 mMol) 1-/4-Chlor-2-[(2-fluorphenyl)hydroxymethyl]-phenyl/-3-phthalimidopropin, 2,0 g (30 mMol) Natriumazid und 0,4 ml (6,6 mMol) Essigsäure in 30 ml Dimethylsulfoxid wird in einem Oelbad während 4 Tagen auf 90° erwärmt. Die Mischung wird abgekühlt und mit Wasser verdünnt. Der entstandene Niederschlag wird abfiltriert, wobei man 4-/4-Chlor-2-[(2-fluorphenyl)hydroxymethyl]phenyl/-5-(N-phthalimidomethyl)-2H-1,2,3-triazol als gelben Festkörper erhält. Nach Umkristallisieren einer Probe dieses Materials aus Aether erhält man weissliche Prismen vom Smp. 160-162°.

Beispiel 12

Eine Lösung von 2,67 M Jones-Reagens (5 ml, 13,3 mMol) wird tropfenweise zu einer Lösung von 1,0 g (2,1 mMol) 4-/4-Chlor-2-[(2-fluorphenyl)hydroxymethyl]phenyl/-5-(N-phthalimidomethyl)-2H-1,2,3-triazol in 20 ml Aceton gegeben. Man rührt die Mischung während einer Stunde bei Raumtemperatur und

zerstört anschliessend überschüssiges Jones-Reagens durch Zugabe von Isopropanol. Nach Abdekantieren wird die Acetonlösung im Vakuum eingedampft. Der Rückstand wird in Methylenchlorid gelöst. Diese Lösung wird mit Wasser gewaschen, über Natriumsulfat getrocknet und eingedampft. Nach Behandeln des Rückstandes mit Aether erhält man 4-[4-Chlor-2-(2-fluorbenzoyl)phenyl]-5-[N-phthalimidomethyl]-2H-1,2,3-triazol vom Smp. 133-135° (Schaumbildung), das in allen Belangen mit authentischem Material identisch ist.

Beispiel 13

Eine Mischung aus 0,17 g (1,0 mMol) Palladiumchlorid, 0,5 g (2,0 mMol) Triphenylphosphin, 0,1 g (0,5 mMol) Kupfer-(I)jodid, 15 g (43 mMol) 5-Chlor-2-iodbenzophenon und 60 ml Diäthylamin wird während 20 Minuten bei Raumtemperatur gerührt. In einer Portion versetzt man mit 6,0 g (107 mMol) Propargylalkohol und rührt die erhaltene Mischung während 24 Stunden. Man entfernt das Lösungsmittel im Vakuum und nimmt den Rückstand in Aether auf. Die ätherische Phase wird mit Wasser gewaschen, über Natriumsulfat getrocknet und im Vakuum eingedampft, wobei 3-Hydroxy-1-[4-chlor-2-benzoylphenyl]propin als rotes Oel anfallen.

Eine Mischung aus 10,1 g (36 mMol) 3-Hydroxy-1-[4-chlor-2-benzoylphenyl]propin, 13 g (57 mMol) Benzosäureanhydrid, 13 ml (160 mMol) Pyridin, 50 ml N,N-Dimethylaminopyridin und 250 ml Methylenchlorid wird während 24 Stunden bei Raumtemperatur gerührt. Die Methylenchloridlösung wird mit wässriger Kupfersulfatlösung und Wasser gewaschen, über Natriumsulfat getrocknet und im Vakuum eingedampft, wobei 10 g eines braunen Oeles anfallen. Durch Behandeln des Rückstandes mit einer Mischung aus Aether und Petroläther erhält man 3-Benzoyloxy-1-[4-chlor-2-benzoylphenyl]propin als farblosen Festkörper vom Smp. 66-67°.

Beispiel 14

Eine Mischung aus 5,9 g (16 mMol) 3-Benzoyloxy-1-[4-chlor-2-benzoylphenyl]propin, 5,2 g (80 mMol) Natriumazid und 210 ml N,N-Dimethylformamid wird in einem Oelbad während 30 Stunden auf 80-90° erwärmt. Nach Abkühlen giesst man die Mischung auf 520 ml Eiswasser und schüttelt mit Methylenchlorid aus. Die Methylenchloridlösung wird mit Wasser gewaschen, über Natriumsulfat getrocknet und im Vakuum eingedampft. Durch Kristallisieren des verbleibenden Oeles aus Methylenchlorid/Hexan erhält man leicht bräunliche Kristalle. Durch Umkristallisieren aus Methylenchlorid/Hexan erhält man 4-Azidomethyl-5-[4-chlor-2-(benzoyl)phenyl]-2H-1,2,3-triazol als weissliche Kristalle vom Smp. 128-131°.

Beispiel 15

Eine Mischung aus 3 g (8,9 mMol) 4-Azidomethyl-5-[4-chlor-2-(benzoyl)phenyl]-2H-1,2,3-triazol und etwa 3 Teelöffel Raney Nickel in 150 ml Aethanol wird während einer Stunde in einem Parr-Apparat hydriert (Anfangsdruck: 5 psi). Nach Abfiltrieren des Katalysators wird das Filtrat im Vakuum eingedampft, wobei 2 g einer gummiartigen Masse anfallen. Der Rückstand wird unter Eluieren mit Essigester/Methylenchlorid (1 : 1) an Kieselgel chromatographiert. Der gummiartige Rückstand, den man nach Entfernen des Lösungsmittels erhält, kristallisiert aus wenig Essigester und liefert weisse Kristalle. Durch Umkristallisieren aus Essigester/Petroläther erhält man 8-Chlor-6-phenyl-2H,4H-[1,2,3]triazolo[4,5-d][2]benzazepin als farblose Prismen vom Smp. 203-205°.

Beispiel 16

Eine Mischung aus 76 g (1,1 Mol) Natriumnitrit und 450 ml Schwefelsäure wird auf einem Dampfbad auf ca. 80° erwärmt, bis alles in Lösung ist. Die Lösung wird auf 30° abgekühlt und portionenweise mit 232 g (1,0 Mol) 2-Amino-5-chlorbenzophenon versetzt, so dass die Temperatur zwischen 30 und 40 °C bleibt. Die Mischung wird während einer Stunde gerührt und anschliessend langsam auf eine Mischung aus 3 Liter Eis und Wasser gegeben. Nach Filtrieren über Hy-Flo wird die gerührte Lösung langsam zu einer Lösung von 200 g (1,83 Mol) Natriumtetrafluoroborat in 800 ml Wasser gegeben. Der erhaltene Niederschlag wird abfiltriert und zweimal mit je 100 ml Wasser gewaschen, wobei ein feuchter, weisser Festkörper anfällt.

Das feuchte 2-Benzoyl-4-chlorbenzoldiazoniumtetrafluoroborat wird in 3 Liter Wasser aufgeschlämmt und tropfenweise mit einer Lösung von 332 g (2 Mol) Kaliumjodid in 1 Liter Wasser versetzt. Die Mischung wird während 4 Stunden bei Raumtemperatur gerührt. Man filtriert den erhaltenen Niederschlag ab, und gibt das Rohprodukt zu 1 Liter siedendem Aether, filtriert und trocknet über Natriumsulfat. Die Aetherlösung wird auf 500 ml eingeengt und mit 100 ml Petroläther versetzt. Eine Probe des ausgefallenen 5-Chlor-2-iodbenzophenons wird aus Aether/Petroläther umkristallisiert und liefert hellgelbe Prismen vom Smp. 80-82°.

Beispiel 17

In Analogie zur Herstellung von 5-Chlor-2-jodbenzophenon erhält man 5-Chlor-2′-fluor-2-jodbenzophenon als hellgelbe Prismen vom Smp. 78-81°.

Beispiel 18

In Analogie zur Herstellung von 5-Chlor-2-jodbenzophenon erhält man 2',5-Dichlor-2-jodbenzophenon als hellgelbe Prismen vom Smp. 64-66°.

Beispiel 19

In Analogie zur Herstellung von 5-Chlor-2-jodbenzophenon erhält man 2-Jodbenzophenon als braunes Oel. Eine Probe dieses Materials wird durch Säulenchromatographie gereinigt und liefert 2-Jodbenzophenon als weisse Prismen vom Smp. 29-31°.

Beispiel 20

in Analogie zur Herstellung von 5-Chlor-2-jodbenzophenon erhält man 2'-Chlor-2-jodbenzophenon als schwach gelbe Prismen vom Smp. 62-64°.

Beispiel 21

Eine Mischung aus 0,71 g (4,0 mMol) Palladiumchlorid, 2,1 g (8,0 mMol) Triphenylphosphin, 0,80 g (4,2 mMol) Kupfer(I)jodid, 68,8 g (0,20 Mol) 5-Chlor-2-jodbenzophenon, 200 ml Diäthylamin und 400 ml Methylenchlorid wird bei Raumtemperatur und unter Argon solange gerührt, bis eine klare Lösung entsteht. In einer Portion gibt man 40,0 g (0,22 Mol) N-Propargylphthalimid hinzu und rührt die erhaltene Mischung während 20 Stunden. Nach Entfernen der flüchtigen Anteile im Vakuum wird der Rückstand mit 200 ml Isopropanol behandelt. Das ausgefallene rohe 1-[4-Chlor-2-benzoylphenyl]-3-phthalimidopropin wird abfiltriert und aus Aceton umkristallisiert. Man erhält crèmefarbene Prismen vom Smp. 148-150°.

Beispiel 22

In Analogie zur Herstellung von 1-[4-Chlor-2-benzoylphenyl]-3-phthalimidopropin erhält man 1-[4-Chlor-2-(2-fluorbenzoyl)phenyl]-3-phthalimidopropin als crèmefarbene Prismen vom Smp. 158-161°.

Beispiel 23

In Analogie zur Herstellung von 1-[4-Chlor-2-benzoylphenyl]-3-phthalimidopropin erhält man 1-[4-Chlor-2-(2-chlorbenzoyl)phenyl-3-phthalimidopropin als crèmefarbene Prismen vom Smp. 144-145°.

Beispiel 24

In Analogie zur Herstellung von 1-[4-Chlor-2-benzoylphenyl]-3-phthalimidopropin erhält man 1-[2-(2-Chlorbenzoyl)phenyl]-3-phthalimidopropin als crèmefarbene Prismen vom Smp. 149-150°.

Beispiel 25

In Analogie zur Herstellung von 1-[4-Chlor-2-benzoylphenyl]-3-phthalimidopropin erhält man 1-[2-benzoylphenyl]-3-phthalimidopropin als crèmefarbene Prismen vom Smp. 164-165°.

Beispiel 26

Eine Mischung aus 2,6 g (9,4 mMol) 8-Chlor-6-(2-fluorphenyl)-2H,4H-[1,2,3]triazolo[3,4-d][2]benzazepin, 2,0 ml (21 mMol) Dimethysulfat und 10 ml 3N wässrige Natronlauge in 25 ml Aethanol wird während 5 Stunden bei Raumtemperatur gerührt. Die Reaktionsmischung wird mit Wasser verdünnt, mit 1N Salzsäure neutralisiert und mit Methylenchlorid ausgeschüttelt. Die Methylenchloridlösung wird mit Wasser gewaschen, über Natriumsulfat getrocknet und im Vakuum eingedampft, wobei ein gelbes Oel anfällt. Nach Reinigung durch Säulenchromatographie (Kieselgel, 35 g ; Elutionsmittel : Essigester) erhält man als Hauptkomponente 8-Chlor-6-(2-fluorphenyl)-2-methyl-2H,4H-[1,2,3]triazolo[3,4-d][2]benzazepin (A) als Schaum. Durch Kristallisieren aus Aether/Petroläther erhält man farblose Kristalle vom Smp. 105-107°. Der Verbindung A wurde die Struktur des in 2-Stellung alkylierten Produktes zugeordnet.

Aus einer späteren Fraktion erhält man 8-Chlor-6-(2-fluorphenyl)-3-methyl-3H,4H-[1,2,3]triazolo[3,4-d][2]benzazepin (B) als farbloses Oel, das nach Behandeln mit 2 ml 1M methanolischer Methansulfonsäure feine gelbe Nadeln vom Smp. 255-257°. ergibt. Der Verbindung B wurde die Struktur des in 3-Stellung alkylierten Produktes zugeordnet.

Beispiel 27

Eine Mischung aus 54 g (0,15 Mol) 5-Chlor-2'-fluor-2-jodbenzophenon und 28,4 g (0,75 Mol)

Natriumborhydrid in 1 000 ml Aethanol wird während 15 Minuten bei 0° gerührt. Die Lösung wird mit Wasser verdünnt und mit Aether ausgeschüttelt. Die ätherische Phase wird mit Wasser gewaschen, über Natriumsulfat getrocknet und im Vakuum eingedampft, wobei 5-Chlor-2'-fluor-2-jodbenzhydrol als dunkelgelbes Oel anfällt.

Beispiel 28

In Analogie zur Herstellung von 1-[4-Chlor-2-benzoylphenyl]-3-phthalimidopropin erhält man 1-/4-Chlor-2-[(2-fluorphenyl)hydroxymethyl]phenyl/-3-phthalimidopropin als schwach gelbe Prismen vom Smp. 158-160°.

Beispiel A

Herstellung von Tabletten (Nassgranulierung)

| | Bestandteile | mg/Tablette | mg/Tablette | mg/Tablette | mg/Tablette |
|---|---|---|---|---|---|
| 1. | 8-chlor-6-(2-chlor-phenyl)-2H,4H-[1,2,3]triazolo[4,5-d][2]benzazepin<br>oder<br>8-chlor-6-phenyl-2H,4H-[1,2,3]triazo-lo[4,5-d][2]benzazepin | 1 | 5 | 10 | 25 |
| 2. | Lactose | 202 | 232 | 261 | 280 |
| 3. | Modifizierte Stärke | 25 | 35 | 45 | 55 |
| 4. | Vorgelatinisierte Stärke | 20 | 25 | 30 | 35 |
| 5. | Destilliertes Wasser q.s. | - | - | - | - |
| 6. | Magnesium-stearat | 2 | 3 | 4 | 5 |
| | Tablettengewicht | 250 mg | 300 mg | 350 mg | 400 mg |

Man mischt die Bestandteile 1-4 in einem geeigneten Mixer und granuliert mit genügend Wasser um eine gute Konsistenz zu erhalten. Nach dem Mahlen wird in einem geeigneten Ofen getrocknet. Anschliessend mahlt und vermischt man während 3 Minuten mit Magnesiumstearat. Schliesslich wird das Gemisch maschinell zu Tabletten entsprechender Grösse verpresst.

(Siehe Tabellen Seite 15 f.)

Beispiel B

Herstellung von Tabletten (Direkte Verpressung)

| Bestandteile | mg/Tablette | mg/Tablette | mg/Tablette | mg/Tablette |
|---|---|---|---|---|
| 1. 8-chlor-6-(2-chlor-phenyl)-2H,4H-[1,2,3]triazolo[4,5-d][2] benzazepin oder 8-chlor-6-phenyl-2H,4H-[1,2,3]triazo-lo[4,5-d][2] benzazepin | 1 | 5 | 10 | 25 |
| 2. Lactose | 221 | 217 | 212 | 181 |
| 3. Avicel | 45 | 45 | 45 | 55 |
| 4. Stärke (direkt verpressbar) | 30 | 30 | 30 | 35 |
| 5. Magnesium-stearat | 3 | 3 | 3 | 4 |
| Tablettengewicht | 300 mg | 300 mg | 300 mg | 300 mg |

Man mischt Bestandteil 1 mit einer äquivalenten Menge Lactose. Anschliessend wird diese Mischung mit den Bestandteilen 3, 4 und dem Rest von Bestandteil 2 gut vermischt. Nach Zugabe von Magnesiumstearat mischt man während 3 Minuten. Die Mischung wird schliesslich maschinell zu Tabletten entsprechender Grösse verpresst.

Beispiel C

Herstellung von Kapseln

| Bestandteile | mg/ Kapsel | mg/ Kapsel | mg/ Kapsel | mg/ Kapsel |
|---|---|---|---|---|
| 1. 8-chlor-6-(2-chlor-phenyl)-2H,4H-[1,2,3]triazolo[4,5-d][2] benzazepin oder 8-chlor-6-phenyl-2H,4H-[1,2,3]triazolo[4,5-d] [2]benzazepin | 1 | 5 | 10 | 25 |
| 2. Lactose | 203 | 293,5 | 328 | 372,5 |
| 3. Stärke | 30 | 35 | 40 | 30 |
| 4. Talk | 15 | 15 | 20 | 20 |
| 5. Aerosol OT | 1 | 1,5 | 2 | 2,5 |
| Füllgewicht der Kapseln | 250 mg | 350 mg | 400 mg | 450 mg |

Die Bestandteile 1, 2, 3 und 5 werden in einem geeigneten Mixer gut vermischt. Nach Zugabe von Talk wird wiederum gut vermischt und die Mischung mit einer geeigneten Maschine in Kapseln abgefüllt.

**Ansprüche**

1. Triazolobenzazepine der allgemeinen Formel

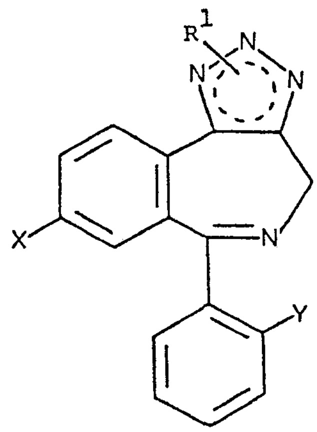

(I)

worin $R^1$ Wasserstoff oder $C_1$-$C_7$ Alkyl, X und Y je Wasserstoff oder Halogen mit einer Atomnummer von höchstens 35 und die gestrichelte Linie 2 zusätzliche Bindungen im Triazolring bedeuten, und pharmazeutisch annehmbare Säureadditionssalze davon.

2. Verbindungen nach Anspruch 1, dadurch gekennzeichnet, dass X Chlor bedeutet.

3. Verbindungen nach Anspruch 1 oder 2, dadurch gekennzeichnet, dass Y Wasserstoff, Chlor oder Fluor bedeutet.

4. Verbindungen nach einem der Ansprüche 1 bis 3, dadurch gekennzeichnet, dass $R^1$ Wasserstoff oder Methyl bedeutet.

5. Verbindungen nach Anspruch 4, dadurch gekennzeichnet, dass $R^1$ Methyl in 2- oder 3-Stellung bedeutet.

6. 8-Chlor-6-phenyl-2H,4H-[1,2,3]triazolo[4,5-d][2]-benzazepin.

7. 8-Chlor-6-(2-fluorphenyl)-2H,4H-[1,2,3]triazolo[4,5-d][2]benzazepin.

8. 8-Chlor-6-(2-chlorphenyl)-2H,4H-[1,2,3]triazolo-[4,5-d][2]benzazepin.

9. Verbindungen der allgemeinen Formel

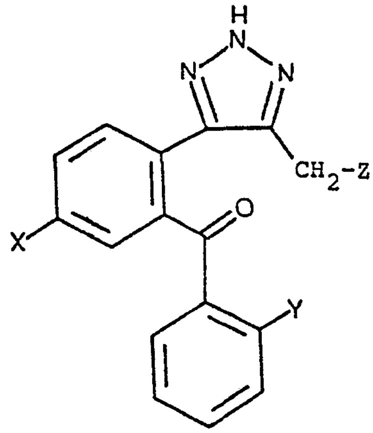

(XIII)

worin X und Y die in Anspruch 1 angegebene Bedeutung besitzen, und Z Amino, Phthalimido oder Azido bedeutet.

10. Verbindungen der allgemeinen Formel

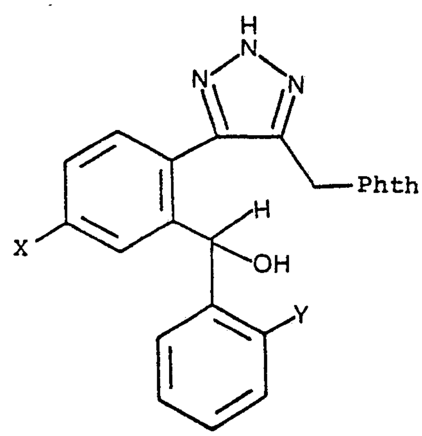

(IX)

worin X und Y die in Anspruch 1 angegebene Bedeutung besitzen, und Phth Phthalimido bedeutet.

11. Verbindungen nach einem der Ansprüche 1 bis 8 zur Verwendung als pharmazeutische Wirkstoffe.

12. Verbindungen nach einem der Ansprüche 1 bis 8 zur Verwendung als anxiolytische, sedative, muskelrelaxierende und anticonvulsive Wirkstoffe.

13. Verfahren zur Herstellung von Verbindungen gemäss einem der Ansprüche 1 bis 8, dadurch gekennzeichnet, dass man eine Verbindung der allgemeinen Formel

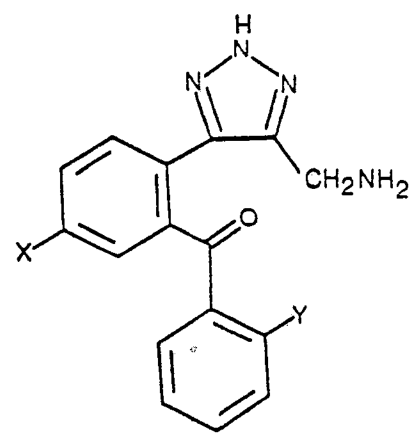

(II)

worin X und Y die in Anspruch 1 angegebene Bedeutung besitzen, cyclisiert, erwünschtenfalls eine erhaltene Verbindung der allgemeinen Formel

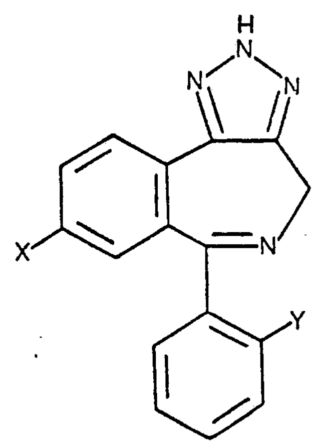

(Ia)

worin X und Y die in Anspruch 1 angegebene Bedeutung besitzen, entsprechend alkyliert, und/oder erwünschtenfalls eine Verbindung der allgemeinen Formel I in ein pharmazeutisch annehmbares Säureadditionssalz überführt.

14. Arzneimittel, enthaltend eine Verbindung nach einem der Ansprüche 1 bis 8.

15. Anxiolytisch, sedativ muskelrelaxierend und anticonvulsiv wirksame Mittel, enthaltend eine Verbindung gemäss einem der Ansprüche 1 bis 8.

**Claims**

1. Triazolobenzazepines of the general formula

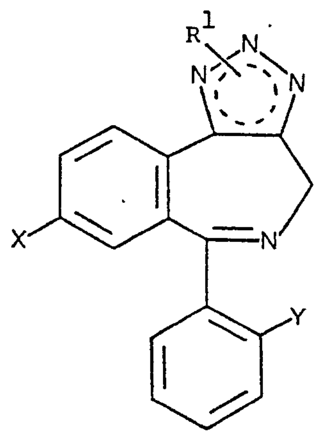

(I)

wherein $R^1$ signifies hydrogen or $C_1$-$C_7$ alkyl, X and Y each signify hydrogen or halogen with an atomic number of at most 35 and the dotted line signifies 2 additional bonds in the triazole ring, and pharmaceutically acceptable acid addition salts thereof.

2. Compounds according to claim 1, characterized in that X signifies chlorine.

3. Compounds according to claim 1 or 2, characterized in that Y signifies hydrogen, chlorine or fluorine.

4. Compounds according to any one of claims 1 to 3, characterized in that $R^1$ signifies hydrogen or methyl.

5. Compounds according to claim 4, characterized in that $R^1$ signifies methyl in the 2- or 3-position.

6. 8-Chloro-6-phenyl-2H,4H-[1,2,3]triazolo[4,5-d]-[2]benzazepine.

7. 8-Chloro-6-(2-fluorophenyl)-2H,4H-[1,2,3]-triazolo[4,5-d][2]benzazepine.

8. 8-Chloro-6-(2-chlorophenyl)-2H,4H-[1,2,3]triazolo[4,5-d]benzazepine.

9. Compounds of the general formula

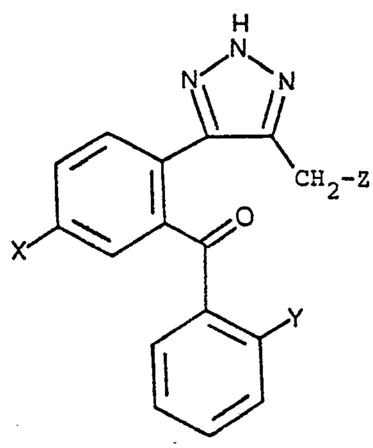

(XIII)

wherein X and Y have the significance given in claim 1, and Z signifies amino, phthalimido or azido.

10. Compounds of the general formula

(Siehe Figur Seite 19 f.)

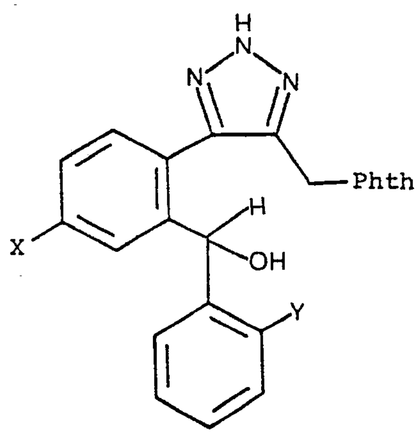

(IX)

wherein X and Y have the significance given in claim 1, and Phth signifies phthalimido.

11. Compounds according to any one of claims 1 to 8 for use as pharmaceutically active substances.

12. Compounds according to any one of claims 1 to 8 for use as anxiolytic, sedative, muscle relaxant and anticonvulsant active substances.

13. A process for the manufacture of compounds in accordance with any one of claims 1 to 8, characterized by cyclizing a compound of the general formula

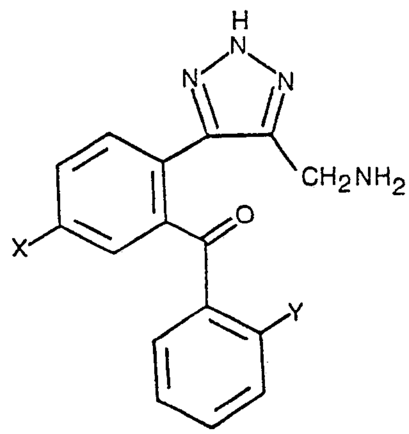

(II)

wherein X and Y have the significance given in claim 1, if desired appropriately alkylating a resulting compound of the general formula

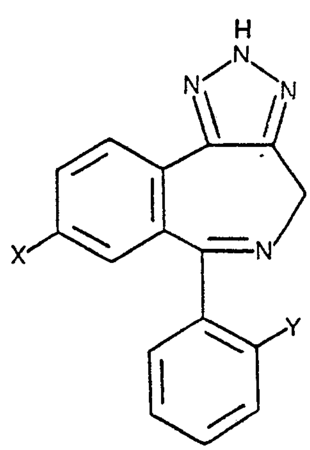

(Ia)

wherein X and Y have the significance given in claim 1, and/or, if desired, converting a compound of general formula I into a pharmaceutically acceptable acid addition salt.

14. Medicaments, containing a compound according to any one of claims 1 to 8.

15. Medicaments, having anxiolytic, sedative, muscle relaxant and anticonvulsant activity, containing a compound according to any one of claims 1 to 8.

**Revendications**

1. Triazolobenzazépines de formule générale

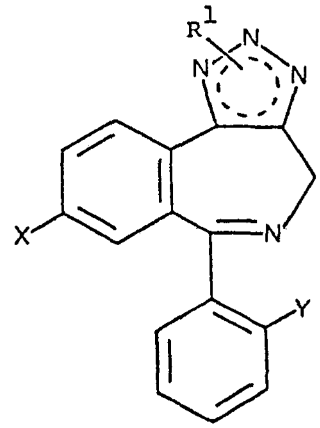

(I)

dans laquelle $R^1$ représente l'hydrogène ou un groupe alkyle en $C_1$-$C_7$, X et Y représentent chacun l'hydrogène ou un halogène de numéro atomique 35 au maximum et le trait interrompu représente deux liaisons supplémentaires dans le cycle triazole, et leurs sels d'addition d'acides acceptables pour l'usage pharmaceutique.

2. Composés selon la revendication 1, caractérisés en ce que X représente le chlore.

3. Composés selon la revendication 1 ou 2, caractérisés en ce que Y représente l'hydrogène, le chlore ou le fluor.

4. Composés selon l'une des revendications 1 à 3, caractérisés en ce que $R^1$ représente l'hydrogène ou le groupe méthyle.

5. Composés selon la revendication 4, caractérisés en ce que $R^1$ représente un groupe méthyle en position 2 ou 3.

6. La 8-chloro-6-phényl-2H,4H-[1,2,3]triazolo[4,5-d][2]benzazépine.

7. La 8-chloro-6-(2-fluorophényl)-2H,4H-[1,2,3]triazolo[4,5-d][2]benzazépine.

8. La 8-chloro-6-(2-chlorophényl)-2H,4H-[1,2,3]triazolo[4,5-d][2]benzazépine.

9. Composés de formule générale

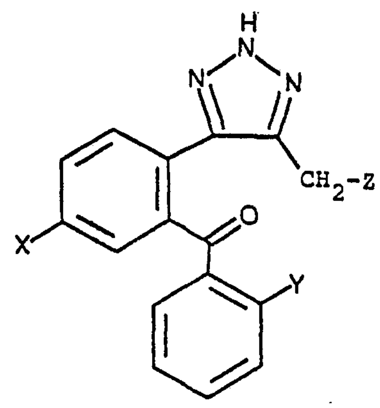

(XIII)

dans laquelle X et Y ont les significations indiquées dans la revendication 1 et Z représente un groupe amino, phtalimido ou azido.

10. Composés de formule générale

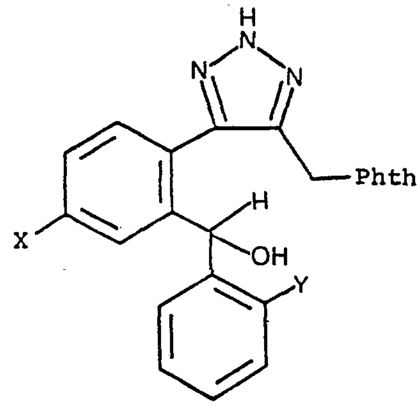

(IX)

dans laquelle X et Y ont les significations indiquées dans la revendication 1, et Phth représente le groupe phtalimido.

11. Composés selon l'une des revendications 1 à 8, pour l'utilisation en tant que substances actives pharmaceutiques.

12. Composés selon l'une des revendications 1 à 8, pour l'utilisation en tant que substances actives anxiolytiques, sédatives, myorelaxantes et anticonvulsives.

13. Procédé de préparation des composés selon l'une des revendications 1 à 8, caractérisé en ce que l'on cyclise un composé de formule générale

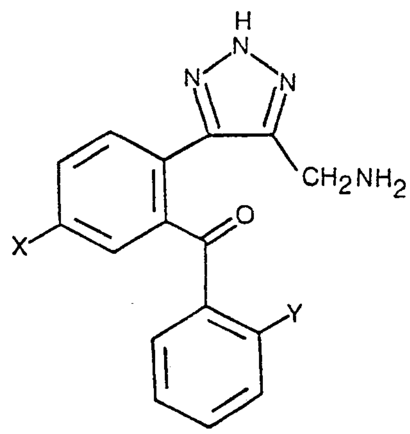

(II)

dans laquelle X et Y ont les significations indiquées dans la revendication 1, si on le désire, on soumet un composé obtenu répondant à la formule générale

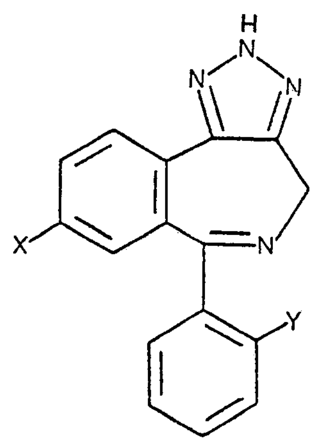

(Ia)

dans laquelle X et Y ont les significations indiquées dans la revendication 1, à l'alkylation correspondante, et/ou si on le désire, on convertit un composé de formule générale I en un sel d'addition d'acide acceptable pour l'usage pharmaceutique.

14. Médicaments contenant un composé selon l'une des revendications 1 à 8.

15. Médicaments anxiolytiques, sédatives, myorelaxantes et anti-convulsives, contenant un composé selon l'une des revendications 1 à 8.